# EUROPÄISCHE PATENTSCHRIFT

(11) **EP 1 613 220 B1**
(45) Veröffentlichungstag und Bekanntmachung des Hinweises auf die Patenterteilung: **06.06.2007**
(21) Anmeldenummer: 04714307.8
(22) Anmeldetag: 25.02.2004
(51) Int. Cl.: A61B 17/16

(54) **RASPELAUFSATZ FÜR EIN MOTORGETRIEBENES CHIRURGISCHES HANDGERÄT**
RASP ATTACHMENT FOR A MOTOR-DRIVEN SURGICAL HAND-HELD DEVICE
EMBOUT SOUS FORME DE RAPE POUR APPAREIL A MAIN CHIRURGICAL ENTRAINE PAR MOTEUR

(30) Priorität: 11.04.2003 DE 20305915 U; 11.04.2003 DE 10316781
(43) Veröffentlichungstag der Anmeldung: 11.01.2006
(73) Patentinhaber: Nolde, Martin, 89407 Dillingen (DE)
(72) Erfinder: Nolde, Martin, 89407 Dillingen (DE)
(74) Vertreter: Schoppe, Fritz
(86) Internationale Anmeldenummer: PCT/EP2004/001863
(87) Internationale Veröffentlichungsnummer: WO 2004/089226

(56) Entgegenhaltungen:
- EP-A- 0 634 145
- EP-A- 0 788 773
- WO-A-93/15665
- US-A- 6 048 345

## Beschreibung

Die vorliegende Erfindung bezieht sich auf einen Raspelaufsatz für ein motorgetriebenes chirurgisches Handgerät zum Abtragen von Knochen oder anderem festen Gewebe.

Bei zahlreichen chirurgischen Eingriffen wird Knochensubstanz durch Feilen, Raspeln, Hobeln oder Fräsen abgetragen, um Knochenoberflächen zu modellieren. Dies ist beispielsweise erforderlich, um eine Engpaßsituation zu beseitigen, ein Einsetzen eines Implantats vorzubereiten oder bei einem vorausgegangenen gröberen Bearbeitungsschritt entstandene Oberflächenstrukturen nachzubearbeiten. Nachfolgend wird ein Beispiel aus der Schulterchirurgie beschrieben.

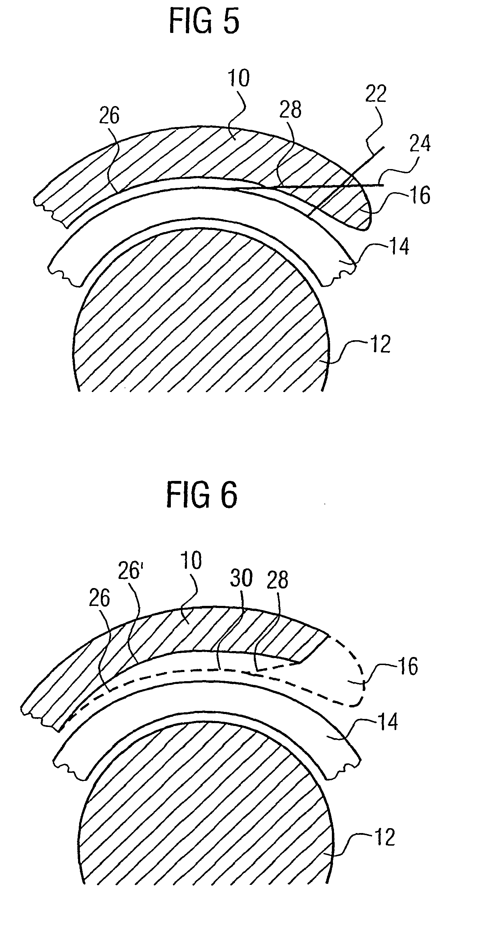

An der Schulter des Menschen führt sehr häufig eine anatomische Variante des Schulterdaches zu einer knöchernen Engpaßsituation, die nicht nur im englischen Sprachraum als Impingement-Syndrom bezeichnet wird. Diese Engpaßsituation führt meist im mittleren Lebensalter zu chronischen bewegungs- und belastungsabhängigen Schmerzen im Schulterbereich. Häufig hat die chronische mechanische Irritation auch eine voranschreitende Schädigung einer unter dem Schulterdach befindlichen Sehnenmanschette, der Rotatorenmanschette, zur Folge. Zur Behandlung des Impingement-Syndroms hat sich weltweit die Erweiterung des Gleitraums unter dem Schulterdach durchgesetzt. Die Fig. 5 und 6 zeigen schematische Darstellungen des Schulterdachs im Längsschnitt bzw. Sagittalschnitt in zwei verschiedenen Behandlungsstadien.

Fig. 5 zeigt das Akromion bzw. Schulterdach 10, den Humeruskopf 12 und die dazwischenliegende Rotatorenmanschette 14 im ursprünglichen Zustand. Der äußere Rand 16 des Schulterdaches 10 ist zu weit heruntergezogen, so daß die Bewegung des Schultergelenks bzw. des Humeruskopfes 12 im Schulterdach 10 eingeschränkt ist. Der nachfolgend skizzierte chirurgische Eingriff wird als Akromioplastik bezeichnet.

Bei der klassischen Akromioplastik wird entsprechend der Vorgabe des Erstautors (Neer) mit dem Meißel das Schulterdach verändert. In den letzten Jahren wird überwiegend eine oszillierende Säge für die Bearbeitung des Schulterdaches 10 eingesetzt. Mittels der oszillierenden Säge werden zwei Sägeschnitte 22, 24 ausgeführt, um den überstehenden Rand 16 des Schulterdachs 10 zu entfernen. Mit der Säge sind jedoch nur gerade bzw. ebene Sägeschnitte erzeugbar. An der der Rotatorenmanschette 14 bzw. dem Humeruskopf 12 zugewandten Unterseite 26 des Schulterdachs 10 entsteht deshalb eine Kante 28, an der in der Regel ein Grat verbleibt. Dieser Grat wird anschließend mittels einer Handraspel oder einer Kugelfräse abgetragen. Dieses Vorgehen ist jedoch mühsam und wenig gewebeschonend.

Je nach Ausprägung der Abweichung der Form des Schulterdachs 10 von der Idealnorm wird bei der Akromioplastik ferner die Unterfläche bzw. Unterseite 26 des Schulterdachs 10 ausgedünnt, um den Gleitraum des Schultergelenks zu erweitern. Der Zustand des Schulterdachs 10 nach der Akromioplastik ist in Fig. 6 gezeigt. Unterbrochene Linien deuten die Kontur des Schulterdachs 10 vor dem Eingriff sowie vor dem Ausdünnen an. Der Rand 16 des Schulterdachs 10 wurde durch die Sägeschnitte 22, 24 entfernt, an der Unterseite 26 des Schulterdachs 10 wurde eine Schicht 30 entfernt, um eine neue Unterseite 26' des Schulterdachs 10 zu bilden.

Der in Fig. 6 dargestellte Zustand stellt einen Idealzustand dar. In der Regel ist eine konkave Formgebung der Unterfläche 26 des Schulterdachs 10 mit der Handraspel jedoch nicht erreichbar. Ein weiterer Nachteil des Stands der Technik besteht, wie bereits angedeutet, darin, daß die Verwendung einer Handraspel oder einer Kugelfräse zur Bearbeitung des Schulterdachs 10 mühsam und wenig gewebeschonend ist.

Aus der US 6048345 A ist ein medizinisches Gerät in Form einer Feile zum Abtragen und Formen von harten Körpermaterialien bekannt auf dem der Oberbegriff des Anspruchs 1 basiert. Ein Feilenkopf, der unterschiedliche Formen aufweisen kann, ist über einen Armabschnitt mit einer Einrichtung zum Hin- und Herbewegen des Feilenkopfs verbunden.

Aus der WO 93/15665 A ist ein chirurgisches Instrument bekannt, das eine Antriebseinrichtung zum Hin- und Herbewegen einer Schneideeinrichtung, die eine Raspelfläche aufweist, umfasst.

Die EP 0788773 A1 offenbart ein Werkzeug zum Implantieren einer Endoprothese im Hohlraum eines menschlichen Knochens. Das Werkzeug enthält einen Raspelkörper, welcher hinsichtlich seiner Form dem im Knochenhohlraum zu verankernden Prothesenschaft entspricht. Am proximalen Ende des Raspelkörpers ist ein Handgriff lösbar befestigt.

Die EP 0634145 A1 offenbart eine Hohlschaftraspel zur Präparation eines Röhrenknochens für die Aufnahme einer stielförmigen Endoprothese. Proximal ist an die Hohlschaftraspel ein Handgriff anbringbar, und ein Längsschlitz verleiht der Hohlschaftsraspel eine federnde Wirkung.

Die Aufgabe der vorliegenden Erfindung besteht darin, ein verbessertes materialabtragendes Werkzeug für chirurgische Anwendungen zu schaffen.

Diese Aufgabe wird durch einen Raspelaufsatz gemäß Anspruch 1 gelöst.

Die vorliegende Erfindung schafft einen Raspelaufsatz für ein motorgetriebenes chirurgisches Handgerät mit einer Befestigungseinrichtung zum Befestigen des Raspelaufsatzes an dem motorgetriebenen chirurgischen Handgerät und einem Raspelbereich, der ausgebildet ist, um bei einer durch das motorgetriebene chirurgische Handgerät bewirkten Hin- und Herbewegung des Raspelaufsatzes Material von einer Oberfläche abzutragen. Der Raspelaufsatz ist zumindest im Raspelbereich gewölbt und elastisch verformbar, wobei die elastische Verformbarkeit in einem von der Befestigungseinrichtung (44) entfernten Bereich höher ist als in einem Bereich nahe der Befestigungseinrichtung (44).

Der vorliegenden Erfindung liegt die Erkenntnis zugrunde, daß ein Operateur ein festes Gewebe, beispielsweise einen Knochen, wesentlich leichter abtragen bzw. modellieren kann, wenn er sich bei diesem Vorgang ausschließlich auf die Anordnung und Ausrichtung der Raspel relativ zu dem zu modellierenden Gewebe, auf den Anpreßdruck und das Spüren der Wechselwirkung zwischen Raspel und Gewebe konzentrieren kann und nicht gleichzeitig Kraft und Energie aufwenden muß und auf eine Gleichmäßigkeit seiner manuellen Raspelbewegungen achten muß. Die vorliegende Erfindung hat in diesem Zusammenhang den Vorteil, daß der Operateur müheloser, wesentlich feinfühliger, gleichmäßiger und gefühlvoller Material abtragen kann als mit einer herkömmlichen Handraspel.

Gemäß der vorliegenden Erfindung wird die eigentliche Raspelarbeit von dem motorgetriebenen chirurgischen Handgerät ausgeführt, deren intrinsische Eigenschaft es ist, daß sie die materialabtragende Hin- und Herbewegung des Raspel- bzw. Feilaufsatzes kinematisch und dynamisch nahezu vollkommen gleichmäßig ausführt. Der Operateur kann sich darauf konzentrieren, durch die Anordnung und Ausrichtung des Raspelaufsatzes relativ zu dem zu modellierenden Gewebe den Ort der Materialabtragung genau zu bestimmen, durch den Anpreßdruck des Raspelaufsatzes bzw. von dessen Raspelbereich auf die abzutragende Oberfläche die Abtragungsrate einzustellen, die Wechselwirkung des Raspelaufsatzes mit der abzutragenden Oberfläche zu beobachten und den Ort der Materialabtragung gegebenenfalls durch eine langsame und gleichmäßige Schwenk- oder Translationsbewegung des Handstücks möglichst kontinuierlich zu verschieben, um beispielsweise Stufenbildungen zu verhindern. Die Beobachtung der Wechselwirkung des Raspelaufsatzes mit der zu bearbeitenden Oberfläche umfaßt vor allem eine Beobachtung der Reibungskraft zwischen dem Raspelbereich und der abzutragenden Oberfläche. Ferner umfaßt sie eine Beobachtung der Rückwirkung auf das motorgetriebene Handstück, die beispielsweise die Frequenz der Hin- und Herbewegung oder ein spürbares Rütteln des Handstücks beeinflußt. Darüber hinaus kann der Operateur beispielsweise über den Raspelaufsatz und das Handstück Stufen und Kanten an der abzutragenden Oberfläche spüren. All dies ermöglicht ein müheloseres, ermüdungsärmeres und deshalb konzentrierteres, präziseres, gewebeschonenderes, schnelleres und damit zeitsparendes Arbeiten.

Der Raspelaufsatz ist zumindest im Raspelbereich in eine oder zwei Richtungen gewölbt, wobei der Raspelbereich sowohl konvex als auch konkav sein kann. Ein gewölbter Raspelbereich hat den Vorteil, daß ohne weiteres gewölbte Oberflächen modelliert werden können und daß das Risiko einer Stufenbildung an einer abzutragenden Oberfläche verringert ist und fast automatisch kontinuierliche bzw. gerundete Übergänge entstehen.

Ferner ist der Raspelaufsatz zumindest im Raspelbereich elastisch, was eine individuelle Anpassung der resezierenden Maßnahme ermöglicht. Insbesondere erlaubt eine Elastizität des Raspelaufsatzes eine Anpassung der Form desselben an die zu bearbeitende Oberfläche allein schon durch die über das Handstück auf den Raspelaufsatz in Richtung auf die zu bearbeitende Oberfläche ausgeübte Kraft. Die Elastizität des Raspelaufsatzes trägt somit ebenfalls zur Bildung stufenfreier, gerundeter Oberflächen mit möglichst kontinuierlichen Übergängen bei und vermeidet beispielsweise eine Gratbildung sicher.

Besonders bevorzugt weist der Raspelaufsatz an seiner Spitze eine höhere Elastizität auf als in einem Bereich nahe dem Handstück. Dies verbessert die Anpassung der Form des Raspelaufsatzes an die zu bearbeitende Oberfläche weiter. Eine optimale Anpassung bzw. eine optimale Anpaßbarkeit des Raspelaufsatzes an die zu bearbeitende Oberfläche wird erreicht, wenn die Elastizität des Raspelaufsatzes von der Befestigungseinrichtung zu seinem von der Befestigungseinrichtung entfernten Ende hin kontinuierlich zunehmend ist.

Vorzugsweise ist der Raspelaufsatz ferner zumindest im Raspelbereich perforiert, um einen Durchtritt von Spülflüssigkeit zu ermöglichen. Eine gleichmäßige Spülung des Raspelbereichs und der in Bearbeitung befindlichen Oberfläche ist wichtig, um abgetragenes Material zu entfernen, und damit sich die Raspel nicht zusetzt, sondern ihre materialabtragende Wirkung erhalten bleibt. Ferner ist eine Spülung wichtig, um die in Bearbeitung befindliche Oberfläche und das umgebende Gewebe zu kühlen bzw. zu temperieren, um eine übermäßige Erwärmung zu vermeiden, die eine Schädigung des Gewebes zur Folge hätte. Vorzugsweise ist der Raspelaufsatz länglich, wobei die Befestigungseinrichtung an einem Ende und der Raspelbereich am anderen Ende des Raspelaufsatzes angeordnet sind. Ferner ist der Raspelaufsatz vorzugsweise so ausgebildet, daß er von dem motorgetriebenen chirurgischen Handstück um eine Achse nahe der Befestigungseinrichtung hin- und hergeschwenkt wird, so daß der Raspelbereich entlang eines Bogensegments eine oszillierende Bewegung ausführt. Dies ermöglicht ein gleichmäßiges und abhängig von der Amplitude der Oszillation flächiges oder auch lokales Abtragen von Material. Ein Raspelbereich im Sinne der vorliegenden Erfindung ist auch ein feiner Raspelbereich bzw. Feilbereich. Ferner umfaßt der Begriff "Raspelbereich" gemäß der vorliegenden Erfindung neben einem Feilbereich auch einen Hobelbereich mit einer oder mehreren Kanten bzw. Schneiden, die senkrecht zur Bewegungsrichtung des Raspelbereichs angeordnet sind.

Bevorzugte Weiterbildungen sind in den Unteransprüchen definiert.

Bevorzugte Ausführungsbeispiele der vorliegenden Erfindung werden nachfolgend mit Bezug auf die beiliegenden Zeichnungen näher erläutert. Es zeigen:
- Fig. 1A bis 1C: schematische Ansichten eines Raspelaufsatzes gemäß einem bevorzugten Ausführungsbeispiel der vorliegenden Erfindung aus drei Richtungen;
- Fig. 2: eine schematische Darstellung eines Raspelaufsatzes gemäß der vorliegenden Erfindung mit einem motorgetriebenen chirurgischen Handgerät;
- Fig. 3 und 4: schematische Darstellungen eines Operationsgebiets mit einem Raspelaufsatz gemäß der vorliegenden Erfindung; und
- Fig. 5 und 6: schematische Darstellungen eines Operationsgebiets vor bzw. nach einer Akromioplastik.

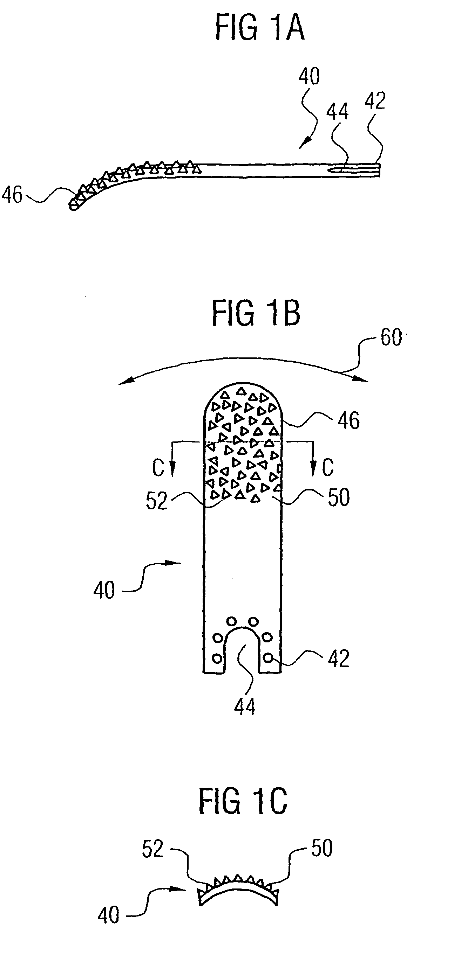

Die Fig. 1A, 1B, 1C zeigen einen Raspelaufsatz 40 gemäß einem bevorzugten Ausführungsbeispiel der vorliegenden Erfindung aus drei verschiedenen Perspektiven. Der Raspelaufsatz 40 hat näherungsweise die Form eines leicht gewölbten, länglichen Plättchens bzw. Blechstreifens. Fig. 1A zeigt den Raspelaufsatz 40 in einer Seitenansicht bzw. aus einer Richtung senkrecht zu der Richtung, in der der Raspelaufsatz 40 seine größte Ausdehnung aufweist, und näherungsweise parallel zu der Richtung, in der der Raspelaufsatz 40 seine zweitgrößte Ausdehnung aufweist. Fig. 1B zeigt den Raspelaufsatz 40 in einer Ansicht von oben bzw. von der Seite, an der der Raspelbereich angeordnet ist, bzw. aus einer Richtung, in der der Raspelaufsatz seine geringste Ausdehnung aufweist. Fig. 1C zeigt eine Darstellung eines Schnitts durch den Raspelaufsatz 40 entlang der Linie C-C in Fig. 1B.

Es ist erkennbar, daß der Raspelaufsatz 40 näherungsweise die Form eines länglichen, rechteckigen Blechstreifens aufweist, an dessen einem Ende 42 eine Befestigungseinrichtung 44 zum Befestigen des Raspelaufsatzes 40 an einem motorgetriebenen chirurgischen Handstück angeordnet ist. Das andere Ende 46 des Raspelaufsatzes 40 ist abgerundet. Nahe dem abgerundeten Ende 46 ist ein Raspelbereich 50 angeordnet, in dem aus der Oberfläche des Raspelaufsatzes 40 eine oder mehrere, vorzugsweise eine Vielzahl von kleinen Kegeln, Pyramiden, Stegen oder anderen konvexen Elementen 52 hervorstehen. Jedes konvexe Element 52 weist eine oder mehrere (vorzugsweise scharfe) Kanten auf, die so ausgebildet sind, daß sie in Eingriff mit einer Oberfläche, entlang der der Raspelbereich 50 bewegt wird, von dieser Material abtragen. Der Raspelbereich 50 ist insofern dem Raspelbereich einer herkömmlichen Handraspel oder Handfeile ähnlich.

In Fig. 1A ist erkennbar, daß der Raspelaufsatz 40 insbesondere im Bereich des Raspelbereichs 50 eine Längswölbung aufweist. In Fig. 1C ist erkennbar, daß der Raspelaufsatz 40 ferner zumindest im Bereich des Raspelbereichs 50 auch eine Wölbung in Querrichtung aufweist. Der Raspelbereich 50 ist in diesem Ausführungsbeispiel an der konvexen Seite der Wölbung des Raspelaufsatzes 40 angeordnet.

Der Raspelaufsatz 40 ist ausgebildet, um vorzugsweise an einem motorgetriebenen chirurgischen Handgerät betrieben zu werden, das den Raspelaufsatz 40 um eine Achse hin- und herschwenkt, die nahe an der Befestigungseinrichtung 44 angeordnet ist. Der Pfeil 60 in Fig. 1B deutet diese oszillierende Schwenkbewegung an.

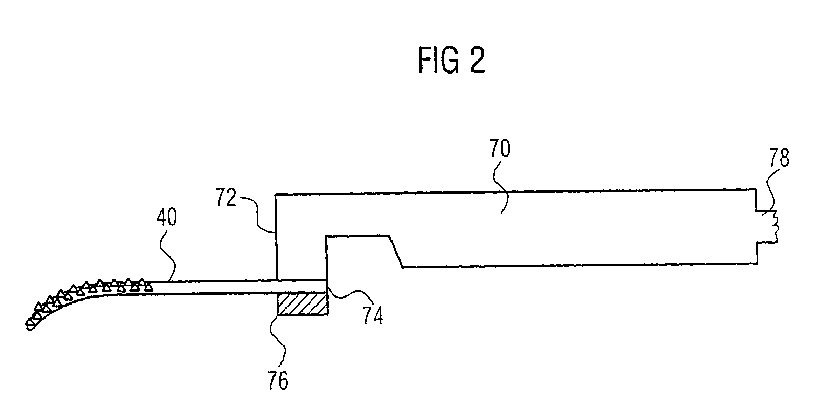

Fig. 2 ist eine schematische Seitenansicht des in den Fig. 1A, 1B dargestellten Raspelaufsatzes 40 zusammen mit einem motorgetriebenen chirurgischen Handstück 70, an dem der Raspelaufsatz 40 angebracht ist. Das motorgetriebene chirurgische Handstück 70 hat eine längliche ergonomisch optimierte Form, die ein sicheres, kraftschlüssiges und ermüdungsfreies Halten mit einer oder mit zwei Händen ermöglicht. An einem vorderen Ende 72 weist das motorgetriebene chirurgische Handstück 70 eine Aufnahme 74 zum Aufnehmen der Befestigungseinrichtung 44 des Raspelaufsatzes 40 auf, die eine kraftschlüssige Verbindung mit der Befestigungseinrichtung 44 ermöglicht. Ein Sicherungselement 76, beispielsweise eine Rändelschraube, ist vorgesehen und ausgebildet, um die Befestigungseinrichtung 44 des Raspelaufsatzes 40 zuverlässig an der Aufnahme 74 zu halten.

Über einen oder mehrere Schläuche oder ein Kabel 78 wird das Handstück 70 mit Druckluft oder einem anderen unter Druck stehenden Fluid bzw. mit elektrischer Leistung versorgt. Ein im Inneren des motorgetriebenen chirurgischen Handstücks 70 angetriebener pneumatischer bzw. hydraulischer bzw. elektrischer Motor dreht die Aufnahme 74 hin und her. Dadurch wird der Raspelbereich 50 des Raspelaufsatzes 40 wie in Fig. 1B durch den Pfeil 60 dargestellt kreisbogenförmig hin- und herbewegt. Anders ausgedrückt wird der Raspelbereich 50 senkrecht zur Längserstreckung des Raspelaufsatzes 40 hin- und herbewegt.

Alternativ ist das Handstück 70 so ausgebildet, daß es gleichzeitig mit oder auch umschaltbar anstelle der Hin- und Herbewegung senkrecht zu der Längserstreckung des Raspelaufsatzes 40 eine radiale Hin- und Herbewegung des Raspelaufsatzes bewirkt. Eine radiale Bewegung ist dabei eine Bewegung parallel zur Längsausdehnung des Raspelaufsatzes 40. Beide Bewegungen weisen, sofern sie gleichzeitig ausgeführt werden, gleich große oder verschiedene Amplituden und Frequenzen auf.

Sowohl das motorgetriebene chirurgische Handstück 70 als auch der Raspelaufsatz 40 sind vorzugsweise sterilisierbar. Alternativ ist der Raspelaufsatz 40 für einen einmaligen Gebrauch vorgesehen, nach dem er weggeworfen bzw. entsorgt wird.

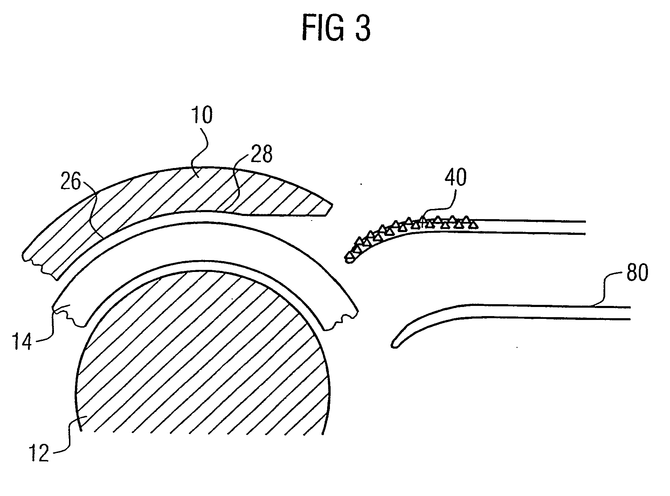

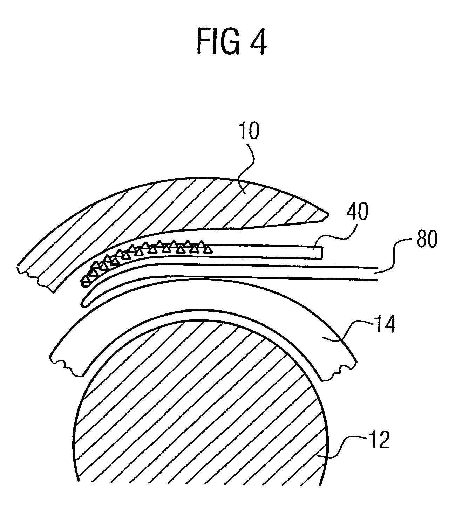

Die Fig. 3 und 4 zeigen schematische Schnittdarstellungen eines Operationsgebiets und des Einsatzes des erfindungsgemäßen Raspelaufsatzes. Beispielhaft ist wiederum die bereits eingangs beschriebene Akromioplastik zur Behandlung des Impingement-Syndroms dargestellt. In Fig. 3 ist das Schulterdach 10 in dem Zustand nach Ausführung der beiden bereits eingangs anhand der Fig. 5 und 6 beschriebenen ebenen Sägeschnitten dargestellt. Zunächst wird ein biegsamer Metallspatel (Düsseldorfer Spatel), wie er in der Abdominalchirurgie eingesetzt wird, zum Schutz der Rotatorenmanschette 14 zwischen dieser und dem Schulterdach 10 plaziert. Durch diesen Spatel kann gleichzeitig der Gelenkkopf bzw. Humeruskopf 12 mit der Rotatorenmanschette 14 nach unten gedrückt bzw. von dem Schulterdach 10 abgerückt werden. Dadurch wird zusätzlicher Arbeitsraum zwischen der Rotatorenmanschette 14 und dem Schulterdach 10 geschaffen. Nach dem Plazieren des Spatels 80 wird der Raspelaufsatz 40 zwischen dem Spatel 80 und dem Schulterdach 10 eingeführt.

Der so erreichte Zustand ist in Fig. 4 dargestellt. Nun wird durch die oszillierende Bewegung des Raspelbereichs 50 des Raspelaufsatzes 40 entlang der Unterseite 26 des Schulterdaches 10 dieses abgetragen, um den bereits eingangs anhand der Fig. 6 dargestellten Zustand zu erreichen. Durch den Einsatz des oszillierenden Raspelaufsatzes 40 mit dem gewölbten Raspelbereich 50 werden in einem einzigen Arbeitsschritt die Unterfläche 26 des Schulterdaches 10 ausgedünnt, konkav geformt und die Schnittfläche des vorangegangenen Sägeschnittes geglättet bzw. der Grat 28 entfernt.

Der erfindungsgemäße Raspelaufsatz ist außer bei der oben beschriebenen Akromioplastik auch bei anderen Eingriffen im orthopädischen und traumatologischen Bereich verwendbar, bei denen Knochen oder auch anderes festes Gewebe abgetragen bzw. modelliert wird. Ein weiteres Beispiel für eine Anwendung des erfindungsgemäßen Raspelaufsatzes ist die moderne Vorfußchirurgie, für die der Raspelaufsatz und insbesondere der Raspelbereich entsprechend auszulegen bzw. Form, Größe, Wölbung, Elastizität und Rauheit des Raspelbereichs 50 zu optimieren sind. Für diese und andere operative Maßnahmen ist die Raspeloberfläche bzw. der Raspelbereich entweder wie oben anhand der Figuren dargestellt an einer konvexen Oberfläche oder aber alternativ an einer konkaven Oberfläche des Raspelaufsatzes plaziert.

Weitere Einsatzgebiete sind kieferchirurgische, handchirurgische, neurochirurgische sowie plastische und wiederherstellungschirurgische Eingriffe.

Wie bereits oben beschrieben, ist ein kontinuierliches Spülen des Operationsgebiets zum Kühlen desselben und zum Entfernen des entstehenden Abraumes bzw. des abgetragenen Materials (beispielsweise Knochenmehl und Knochenmarkszellen) während des Materialabtragungsvorgangs bzw. Fräsvorgangs wichtig. Um das Spülen des Operationsgebiets und insbesondere eine Umspülung des Raspelaufsatzes und seines Raspelbereichs zu begünstigen, ist der Raspelaufsatz und insbesondere dessen Raspelbereich vorzugsweise mit einer Perforation bzw. mit einem oder mehreren Löchern, Schlitzen oder andersartigen Öffnungen versehen, die einen Durchtritt der Spülflüssigkeit von einer Seite des Raspelaufsatzes auf dessen andere Seite ermöglichen. Die konvexen Elemente 52 und die Öffnungen können beispielsweise dadurch gebildet werden, indem U-förmige oder V-förmige Schnitte in ein den Raspelaufsatz 40 bildendes Blech gestanzt, gefräst oder geätzt werden, und die dreieckigen, viereckigen oder halbrunden Abschnitte des Blechs, die innerhalb der Schnitte zurückbleiben, hochgebogen werden.

Der Befestigungsbereich des erfindungsgemäßen Raspelaufsatzes ist vorzugsweise entsprechend den internationalen Normen für Befestigungen von chirurgischen Instrumenten an handgetriebenen oder motorgetriebenen Handstücken ausgelegt.

Wie erwähnt ist der erfindungsgemäße Raspelaufsatz elastisch. Die Elastizität des Raspelaufsatzes nimmt von der Befestigungseinrichtung zu dem von der Befestigungseinrichtung abgewandten Ende zu, um eine gute Anpassung des Raspelaufsatzes an die Form der zu bearbeitenden Oberfläche zu erzielen. Vorzugsweise ist die Elastizität in einem vorderen bzw. von der Befestigungseinrichtung entfernten Drittel höher als in einem hinteren bzw. nahe der Befestigungseinrichtung angeordneten Abschnitt. Eine ideale Anpassung bzw. Anpaßbarkeit wird bei einer kontinuierlichen Zunahme der Elastiztät von der Befestigungseinrichtung zu dem von der Befestigungseinrichtung abgewandten Ende erzielt.

Je größer die Elastizität des Raspelaufsatzes ist, desto größer ist seine Anpaßbarkeit und desto besser kann er sich auch an kleine Oberflächenunebenheiten anpassen. Je geringer die Elastizität des Raspelaufsatzes ist, desto mehr Kraft oder Druck kann von dem Raspelaufsatz auf die zu bearbeitende Oberfläche ausgeübt werden und desto glatter kann eine Oberfläche bearbeitet werden. Es ist deshalb für jeden Anwendungsbereich und für jedes Operationsgebiet jeweils ein Kompromiß zwischen Elastizität und Steifheit zu finden. Die jeweils gewählte Elastizität des erfindungsgemäßen Raspelaufsatzes ist dabei ferner von der bevorzugten Arbeitstechnik des Operateurs abhängig.

## Patentansprüche

1. Raspelaufsatz (40) für ein motorgetriebenes chirurgisches Handstück (70) mit:
einer Befestigungseinrichtung (44) zum Befestigen des Raspelaufsatzes (40) an dem motorgetriebenen chirurgischen Handstück (70); und
einem Raspelbereich (50), der ausgebildet ist, um bei einer durch das motorgetriebene chirurgische Handstück (70) bewirkten Hin- und Herbewegung des Raspelaufsatzes (40) Material von einer Oberfläche abzutragen, wobei der Raspelaufsatz zumindest im Raspelbereich gewölbt und elastisch verformbar ist, **dadurch gekennzeichnet daß**
die elastische Verformbarkeit des Raspelaufsatzes in einem von der Befestigungseinrichtung (44.) entfernten Bereich höher ist als in einem Bereich nahe der Befestigungseinrichtung (44).

2. Raspelaufsatz gemäß Anspruch 1, bei dem der Raspelbereich (50) an einem konkaven oder konvexen Oberflächenabschnitt des Raspelaufsatzes (40) angeordnet ist.

3. Raspelaufsatz (40) gemäß Anspruch 1 oder 2, dessen elastische Verformbarkeit von der Befestigungseinrichtung (44) zu einem von der Befestigungseinrichtung . (44) entfernten Ende (46) hin kontinuierlich zunimmt.

4. Raspelaufsatz (40) gemäß einem der Ansprüche 1 bis 3 mit einer länglichen Form, wobei die Befestigungseinrichtung (44) an einem Ende (42) des Raspelaufsatzes (40) und der Raspelbereich (50) am gegenüberliegenden Ende (46) des Raspelaufsatzes (40) angeordnet sind.

5. Raspelaufsatz (40) gemäß Anspruch 4, der ausgebildet ist, um bei einer kreisbogensegmentförmigen Bewegung des Raspelbereichs (50) Material von der Oberfläche abzutragen.

6. Raspelaufsatz (40) gemäß Anspruch 4 oder 5, bei dem der Raspelbereich (50) ferner ausgebildet ist, um bei einer Bewegung des Raspelaufsatzes (40) in Längsrichtung Material von der Oberfläche abzutragen.

7. Raspelaufsatz (40) gemäß einem der Ansprüche 1 bis 6, ferner mit einer Öffnung, die einen Durchtritt einer Spülflüssigkeit durch den Raspelaufsatz (40) ermöglicht.

8. Raspelaufsatz (40) gemäß einem der Ansprüche 1 bis 7, bei der die Befestigungseinrichtung (44) einer internationalen Norm für chirurgische Geräte entspricht.

9. Motorgetriebenes chirurgisches Handstück (70) mit einem Raspelaufsatz (40) gemäß einem der Ansprüche 1 bis 8.

## Claims

1. Rasp attachment (40) for a motor-driven surgical hand-piece (70), comprising:
mounting means (44) for mounting the rasp attachment (40) to the motor-driven surgical hand-piece (70); and
a rasp area (50) which is implemented to take off material from a surface in a reciprocating movement of the rasp attachment (40) caused by the motor-driven surgical hand-piece (70), wherein the rasp attachment is curved and elastically deformable, at least in the rasp area, **characterized in that**
the elastic deformability of the rasp attachment is higher in an area remote from the mounting means (44) than in an area close to the mounting means (44).

2. Rasp attachment according to claim 1, wherein the rasp area (50) is arranged at a concave or a convex surface portion of the rasp attachment (40).

3. Rasp attachment (40) according to claim 1 or 2, whose elastic deformability continuously increases from the mounting means (44) to an end (46) remote from the mounting means (44).

4. Rasp attachment (40) according to one of claims 1 to 3, having an oblong shape, wherein the mounting means (44) is arranged at an end (42) of the rasp attachment (40) and the rasp area (50) is arranged at the opposite end (46) of the rasp attachment (40).

5. Rasp attachment (40) according to claim 4, which is implemented to take off material from the surface in a movement of the rasp area (50) in the shape of a circular arch segment.

6. Rasp attachment (40) according to claim 4 or 5, wherein the rasp area (50) is further implemented to take off material from the surface in a longitudinal direction with a movement of the rasp attachment (40).

7. Rasp attachment (40) according to one of claims 1 to 6, further comprising an opening facilitating a passage of a rinsing liquid through the rasp attachment (40).

8. Rasp attachment (40) according to one of claims 1 to 7, wherein the mounting means (44) corresponds to an international standard for surgical devices.

9. Motor-driven surgical hand-piece (70) comprising a rasp attachment (40) according to one of claims 1 to 8.

## Revendications

1. Embout sous forme de râpe (40) pour pièce à main chirurgicale entraînée par moteur (70), avec:
un moyen de fixation (44) destiné à fixer l'embout sous forme de râpe (40) à la pièce à main chirurgicale entraînée par moteur (70); et
une zone de râpe (50) qui est réalisée de manière à éliminer du matériau d'une surface lors d'un mouvement de va-et-vient de l'embout sous forme de râpe (40) provoqué par la pièce à main chirurgicale entraînée par moteur (70), l'embout sous forme de râpe étant bombé et déformable élastiquement au moins dans la zone de râpe, **caractérisé par le fait que**
l'aptitude à la déformation élastique de l'embout sous forme de râpe est supérieure dans une zone éloignée du moyen de fixation (44) que dans une zone près du moyen de fixation (44).

2. Embout sous forme de râpe selon la revendication 1, dans lequel la zone de râpe (50) est disposée à un segment de surface concave ou convexe de l'embout sous forme de râpe (40).

3. Embout sous forme de râpe (40) selon la revendication 1 ou 2, dont l'aptitude à la déformation élastique augmente en continu du moyen de fixation (44) vers une extrémité (46) éloignée du moyen de fixation (44).

4. Embout sous forme de râpe (40) selon l'une des revendications 1 à 3, de forme oblongue, le moyen de fixation (44) étant disposé à une extrémité (42) de l'embout sous forme de râpe (40) et la zone de râpe (50) à l'extrémité opposée (46) de l'embout sous forme de râpe (40).

5. Embout sous forme de râpe (40) selon la revendication 4, qui est réalisé de manière à éliminer du matériau de la surface lors d'un mouvement en segment d'arc de cercle de la zone de râpe (50).

6. Embout sous forme de râpe (40) selon la revendication 4 ou 5, dans lequel la zone de râpe (50) est par ailleurs réalisée de manière à éliminer du matériau de la surface lors d'un mouvement de l'embout sous forme de râpe (40) dans le sens longitudinal.

7. Embout sous forme de râpe (40) selon l'une des revendications 1 à 6, avec par ailleurs une ouverture permettant un passage d'un liquide de rinçage dans l'embout sous forme de râpe (40).

8. Embout sous forme de râpe (40) selon l'une des revendications 1 à 7, dans lequel le moyen de fixation (44) est conforme à une norme internationale pour appareils chirurgicaux.

9. Pièce à main chirurgicale entraînée par moteur (70) avec un embout sous forme de râpe (40) selon l'une des revendications 1 à 8.
